# EUROPEAN PATENT SPECIFICATION

(11) **EP 4 076 553 B1**
(45) Date of publication and mention of the grant of the patent: **11.02.2026**
(21) Application number: 20835759.0
(22) Date of filing: 17.12.2020
(51) Int. Cl.: A61L 27/20, A61L 27/26, A61L 27/52, A61L 27/54, A61K 31/728, A61P 19/02, A61K 31/685, A61K 31/737

(54) **SYNOVIAL FLUID SUBSTITUTES**
SUBSTITUTE DER SYNOVIALFLÜSSIGKEIT
SUBSTITUTS DE LIQUIDE SYNOVIAL

(30) Priority: 17.12.2019 IT 201900024214
(43) Date of publication of application: 26.10.2022
(73) Proprietor: IBSA INSTITUT BIOCHIMIQUE SA, 6912 Lugano (CH)
(72) Inventor: CICOGNANI, Marta, 6900 LUGANO (CH); GIORI, Andrea Maria, 6900 LUGANO (CH); VECCHI, Gabriele, 6900 LUGANO (CH)
(74) Representative: Bianchetti & Minoja with Trevisan & Cuonzo IPS SRL
(86) International application number: PCT/EP2020/086644
(87) International publication number: WO 2021/122895

(56) References cited:
- WO-A2-2012/032151
- ANTONIETTA STELLAVATO ET AL: "Novel Hybrid Gels Made of High and Low Molecular Weight Hyaluronic Acid Induce Proliferation and Reduce Inflammation in an Osteoarthritis In Vitro Model Based on Human Synoviocytes and Chondrocytes", BIOMED RESEARCH INTERNATIONAL, vol. 2019, 23 April 2019 (2019-04-23), pages 1 - 13, XP055643719, ISSN: 2314-6133, DOI: 10.1155/2019/4328219

## Description

The invention relates to intra-articular compositions comprising low- and high-molecular-weight linear hyaluronic acid or hyaluronate and is s defined in the claims. Due to their rheological properties, the compositions according to the invention are useful for viscosupplementation as synovial fluid substitutes.

### Prior art

Synovial fluid is a viscous solution mainly consisting of hyaluronic acid, proteins (including lubricin), phospholipids (in particular phosphatidylcholine) and ions. Its function is to lubricate and protect the joints, whether at rest or moving. In order to perform this function effectively, synovial fluid has a viscoelastic, non-Newtonian behaviour whereby its viscosity varies in proportion to the force applied. In the absence of movement, or during slow movements, viscous behaviour (measured by viscous modulus G''), and consequently lubrication capacity, prevails. Conversely, when the joint is moving (for example during fast walking or running), elastic behaviour (measured by elastic modulus G'), which is crucial to protect joints under load, prevails.

In the synovial fluid of a healthy individual, the reversal between the two moduli takes place at low frequencies, generally below 1-2 Hz. Said frequency, called the crossover frequency, corresponds, for example, to the changeover from slow walking to running.

The crossover frequency increases with age and with the progression of joint disorders such as osteoarthritis which, when severe, is accompanied by disappearance of the reversal point. In this case the synovial fluid never exhibits predominantly elastic behaviour, even at high frequency, and G' is always less than G".

This modification of the rheological properties of synovial fluid is accompanied by a change in its composition. There is a reduction in the average molecular weight of the hyaluronic acid, which falls from about 6-7 million Da to 1-3 million, and in its concentration, which falls from 2-4 mg/ml to 1-2 mg/ml. This variation is directly connected with the modification in viscoelastic behaviour, mainly due to the polysaccharide component of the fluid. As a further consequence, the dynamic viscosity falls from an average value of a few dozen Pa*s to values close to or lower than 1 Pa*s. Similarly, the value of G' at the frequency of 2.5 Hz falls from about 120 Pa to values lower than 10 Pa (Balazs, E.A. "Viscoelastic properties of hyaluronic acid and biological lubrication." University of Michigan Medical Center Journal (1967): 255-259, and Fam, H., J. T. Bryant, and M. Kontopoulou. "Rheological properties of synovial fluids." Biorheology 44.2 (2007): 59-74).

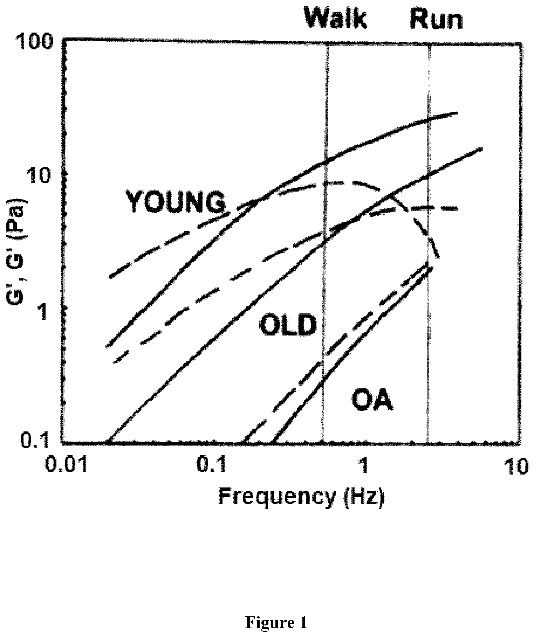

The dynamic moduli of synovial fluid from young, normal and osteoarthritis patients is reported in Figure 1 (taken from Balasz, Disorder of knee, 1982).

Viscosupplementation consists of administering to a patient suffering from joint disorders a liquid which, when introduced directly into the joint cavity, restores the physiological properties of the synovial fluid. For this purpose, the solution used must simulate the viscoelastic behaviour of the synovial fluid of a healthy individual as closely as possible. It must therefore have a similar viscosity and a reversal frequency not exceeding 1-1.5 Hz ( lower than 10rad/s).

Hyaluronic acid is often used for viscosupplementation, usually in the form of sodium hyaluronate, in saline solution. Hyaluronic acid can be used "as is" (linear molecule) or be chemically modified to bond various polysaccharide chains together (crosslinking). The rheology of hyaluronate solutions is directly influenced by molecular weight, concentration and the degree of crosslinking, if any. The higher the molecular weight, the better the viscoelastic behaviour of the solution. Unfortunately, molecular weights similar to the physiological values (6-7 million) are not obtainable by the normal manufacturing techniques, and the hyaluronate used in viscosupplementation has a molecular weight of 1-4 million on average. The main strategies used to circumvent this limitation involve:
1. increasing the concentration, which in the medical devices present on the market can be up to 20 mg/ml (Sinovial^{®} IBSA)
2. increasing the molecular weight by crosslinking, to give gels of chemically modified hyaluronates (Hylan^{®} - US 5099013).

In the first case, such high concentrations (about 10 times the physiological concentration) are accompanied by a considerable increase in viscosity, which is significantly higher than that of synovial fluid, and can lead to difficulties with manufacture and administration.

The same phenomenon arises in the case of crosslinking, which is also usually accompanied by "stiffening" of the three-dimensional structure, with a consequent increase in elastic modulus G', at the expense of viscous modulus G"; in this case a significant alteration (reduction) in crossover frequency is observed compared with synovial fluid, or even the disappearance of the crossover point, where G' is always greater than G'', even at low frequencies. Moreover, crosslinking with chemical agents can increase the presence of impurities and adverse reactions associated with the immunogenicity of the modified hyaluronate, which is less biocompatible.

To obviate the excessive increase in elastic behaviour, US 8524213 proposes mixtures of linear hyaluronate and crosslinked hyaluronate. However, despite a certain improvement in rheological properties, said solution does not eliminate the drawbacks involved in administering a chemically modified hyaluronate, different from the natural one.

Conversely, WO 2012/032151 discloses hybrid cooperative complexes consisting of high-weight HA and a second polysaccharide with a lower molecular weight, which can be another hyaluronate or another chemical species (such as chondroitin sulphate or a maltodextrin). The complex is obtained by heat treatment, leading to an up to 200-fold reduction in dynamic viscosity. The heat treatment also has an impact on the viscoelasticity of the solution, which modifies its rheological profile, shifting the crossover towards high frequencies. Once again, therefore, the preparation obtained is not wholly suitable for viscosupplementation, mainly because the objective of the manufacturing process is not to prepare a mixture of hyaluronate, but to create hybrid cooperative complexes with low viscosity. In addition, viscous modulus G" prevails, even at relatively high frequencies.

A mixture of high and low molecular weight hyaluronic acid useful in the treatment of osteoarthritis is also described by Stellavato et al. in "Novel Hybrid Gels Made of High and Low Molecular Weight Hyaluronic Acid Induce Proliferation and Reduce Inflammation in an Osteoarthritis In Vitro Model Based on Human Synoviocytes and Chondrocytes" Hindawi Biomed Research International (2109), Article ID 4328219, 13 pages.

EP 2 026 821 uses binary mixtures of hyaluronic acid having different molecular weights with the aim of obtaining a formulation with a dynamic viscosity of at least 5 Pa*s (shear rate of 1 sec⁻¹), with a G' of at least 7.5 Pa and a G" of at least 7 Pa at the frequency of 1 Hz. However, as stated in the experimental part (Part A), said result is obtained with mixtures of linear HA (Suplasyn) and crosslinked HA (Hylan). There is therefore still a need to identify compositions of hyaluronic acid which are safe (i.e. based only on the use of native, not chemically modified HA) and effective, i.e. have a rheological profile which simulates that of the synovial fluid of young, healthy individuals.

### Description of the invention

It has now surprisingly been discovered that solutions with rheological behaviour equivalent to that of synovial fluid can be obtained solely by using unmodified linear hyaluronic acid alone, optionally in combination with other minority ingredients already present in synovial fluid (such as proteins, phospholipids and polysaccharides). The hyaluronic acid must be present as a mixture of at least two different molecular weights, to give a solution with controlled rheological properties. No particular procedures are required to prepare said mixtures, still less high-temperature treatments. The mixtures according to the invention exhibit all the characteristics required to replace synovial fluid in patients suffering from joint disorders, and thus constitute the ideal formulations for viscosupplementation.

### Detailed description of the invention

Disclosed is an intra-articular composition comprising at least one low-molecular-weight linear hyaluronic acid or hyaluronate and at least one high-molecular-weight linear hyaluronic acid or hyaluronate, the weight ratio of low-molecular-weight hyaluronate to high-molecular-weight hyaluronate(s) ranging from 1:10 to 10:1.

The compositions of the invention are defined in the appended claims and have a dynamic viscosity at 25 °C ranging between 10 and 60 Pa*s (at 0.01 s⁻¹), a crossover frequency ranging between 1 and 10 rad/s, and a value of the viscous and elastic moduli at the crossover frequency ranging between 40 and 110 Pa.

The low-molecular-weight linear hyaluronic acid or hyaluronate has an average molecular weight Mw ranging between **70** and 90 kDa.

The high-molecular-weight linear hyaluronic acid or hyaluronate has an average molecular weight Mw ranging between 1000 and 3500 kDa.

The weight ratio of low-molecular-weight hyaluronate to high-molecular-weight hyaluronate(s) is ranging from 1:3 to 3:1.

The composition according to the invention comprises low-molecular-weight linear hyaluronic acid or hyaluronate with an average molecular weight Mw ranging between 70 and 90 kDa and high-molecular-weight linear hyaluronic acid or hyaluronate with an average molecular weight Mw ranging between 1000 and 3500 kDa.

Preferably the compositions according to the invention preferably comprise at least two different high-molecular-weight linear hyaluronic acids or hyaluronates.

The MW is determined from intrinsic viscosity η (calculated according to EP) with the Mark-Houwink-Sakurada equation wherein η=KM^{a} (equation reported in standard ASTM F2347-15).

The compositions according to the invention can also contain phospholipids such as phosphatidylcholine and lubricin, and other glycosaminoglycans such as non-sulphated chondroitin.

The low and high-molecular-weight hyaluronic acids usable according to the invention are known and available on the market, for example from Altergon (Italy), HTL (France), Bloomage Freda Biopharm Co. Ltd. (China) and Kewpie (Japan).

The composition according to the invention takes the form of an aqueous solution comprising hyaluronic acids or hyaluronates in concentrations ranging between 1 and 5% w/v.

In addition to hyaluronates and optionally other glycosaminoglycans, phospholipids and proteins such as lubricin, the compositions according to the invention can also contain local anaesthetics, buffering agents and optionally other suitable excipients or active ingredients useful in human and veterinary medicine.

The compositions according to the invention can be administered intra-articularly and intra-synovially for the treatment and prevention of osteoarthritis and correlated disorders.

The doses will depend on various factors, such as the patient's weight and age, and the severity and stage of progress of the disorder, but will in any event be easily determined by healthcare personnel on the basis of experience and the clinical and pre-clinical results obtained with the compositions according to the invention.

The following examples illustrate the invention in greater detail.

### Example 1: rheological evaluation of solutions with increasing concentrations of hyaluronic acid (not part of the invention)

Starting with a sodium hyaluronate obtained by fermentation, having a molecular weight of 1,700 kDa, three aqueous solutions with increasing concentrations are prepared: 16 mg/ml, 21 mg/ml and 30 mg/ml. The preparations are sterilised in the autoclave (121°C for 15 min). The clear solutions with increasing viscosities are analysed with a rheometer equipped with a cone/plate system (Modular Compact Rheometer MCR302, Anton Paar). The measurements are conducted at 25°C. In particular, oscillatory analysis (frequency sweeps) is conducted at frequencies corresponding to different joint movement speeds (0.1-100 rad/s), and rotational analysis to evaluate dynamic viscosity at different shear rates (0.01 - 300 s⁻¹).

The data obtained are summarised in Table 1 below, which also shows the reference values for synovial fluid:

**Table 1**

| sample | crossover | G' and G" at crossover | dynamic viscosity (at 0.01 s⁻¹) |
|---|---|---|---|
| HA 16 mg/ml | 29 rad/s | 90 Pa | 12 Pa*s |
| HA 21 mg/ml | 13 rad/s | 105 Pa | 28 Pa*s |
| HA 30 mg/ml | 9 rad/s | 115 Pa | 90 Pa*s |
| synovial fluid | 1-10 rad/s | 20-80 Pa | 1-40 Pa*s |

An aqueous solution is also prepared at the concentration of 30 mg/ml, starting with a sodium hyaluronate obtained by fermentation having a molecular weight of 300 kDa. The rheological data obtained are summarised in Table 2 below:

**Table 2**

| sample | crossover | G' and G" at crossover | dynamic viscosity (at 0.01 s⁻¹) |
|---|---|---|---|
| HA (300 kDa) 30 mg/ml | -- | -- | 2 Pa*s |
| synovial fluid | 1-10 rad/s | 20-80 Pa | 1-40 Pa*s |

The experimental data demonstrate that at low concentrations of hyaluronate having a molecular weight of 1,700 kDa, the dynamic viscosity value is similar to that of synovial fluid, and the values of G' and G" at crossover are not much higher. Conversely, the crossover frequency is much higher than the physiological value, and is similar to that found in some osteoarticular disorders.

An improvement in crossover frequency can be obtained by increasing the concentration of hyaluronic acid, but this action has the side effect of proportionally increasing the values of the viscous and elastic moduli and the dynamic viscosity, which are very different from the physiological values.

Moreover, the experimental data demonstrate that the use of low-molecular-weight hyaluronate (300 kDa) does not give rise to a formulation with a crossover point, because the viscous component (G'') always prevails over the elastic component (G').

There is therefore no way of wholly simulating the rheological properties of synovial fluid if a single hyaluronic acid is used.

### Example 2: preparation of binary mixtures of hyaluronates with controlled rheological properties

Two binary solutions of sodium hyaluronate obtained by fermentation are prepared, using sodium hyaluronate with a molecular weight of 70 kDa mixed with a hyaluronate having a higher molecular weight. In detail, one solution is prepared using 16 g of hyaluronate with a molecular weight of 1,700 kDa and 16 g of hyaluronate with a molecular weight of 70 kDa dissolved in 1 L of saline solution, and one solution by dissolving 9 g of hyaluronate with a molecular weight of 3,500 kDa and 6 g of hyaluronate with a molecular weight of 70 kDa in 1 L of saline solution.

After terminal sterilisation at a T exceeding 120°C, two clear, viscous solutions are obtained, which are examined at 25°C with a rheometer (Modular Compact Rheometer MCR302, Anton Paar) equipped with a cone/plate system to determine the crossover frequency, the values of the elastic and viscous moduli at crossover, and the dynamic viscosity.

The rheological data of the two solutions, called HA HMW-LMW and HA VHMW-LMW, are summarised in Table 3 below, which also shows the reference values for synovial fluid:

**Table 3**

| sample | crossover | G' and G" at crossover | dynamic viscosity (at 0.01 s⁻¹) |
|---|---|---|---|
| HA VHMW-LMW | 9 rad/s | 23 Pa | 12 Pa*s |
| HA HMW-LMW | 9 rad/s | 79 Pa | 20 Pa*s |
| synovial fluid | 1-10 rad/s | 20-80 Pa | 1-40 Pa*s |

Both solutions exhibit rheological behaviour similar to that of the synovial fluid of a healthy individual: the higher the molecular weight of the high-molecular-weight hyaluronate, the more similar the solution is to synovial fluid.

### Example 3: preparation of ternary mixtures of hyaluronates with controlled rheological properties

5 g of sodium hyaluronate obtained by fermentation, with a molecular weight of 3,500 kDa, 5 g of sodium hyaluronate obtained by fermentation, with a molecular weight of 1,700 kDa, and 5 g of sodium hyaluronate obtained by fermentation, with a molecular weight of 70 kDa, are dissolved in 1 L of saline solution. After sterilisation, a clear viscous solution is obtained, the rheological properties of which are examined with a rheometer (Modular Compact Rheometer MCR302, Anton Paar) equipped with a cone/plate system at 25°C.

The solution, called HA VHMW-HMW-LMW, exhibits a crossover value of 9 rad/s (crossover in healthy individuals: 1-10 rad/sec), a G' and G" value at the crossover frequency of 40 Pa (physiological values in healthy individuals < 100 Pa), and a dynamic viscosity at a low shear rate of 16 Pa*s (in healthy individuals, the dynamic viscosity ranges between 1 and 40 Pa*s).

The rheological behaviour of the solution prepared is therefore identical to that of the synovial fluid of a healthy individual.

### Example 4: comparison of solutions with crossover frequency similar to the physiological frequency

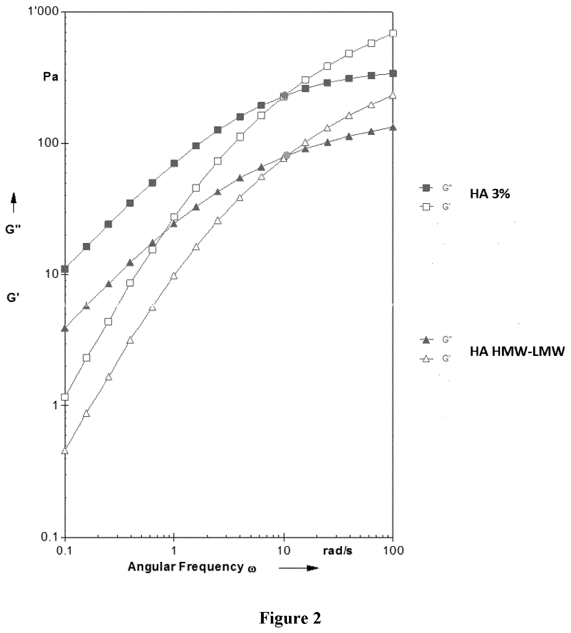

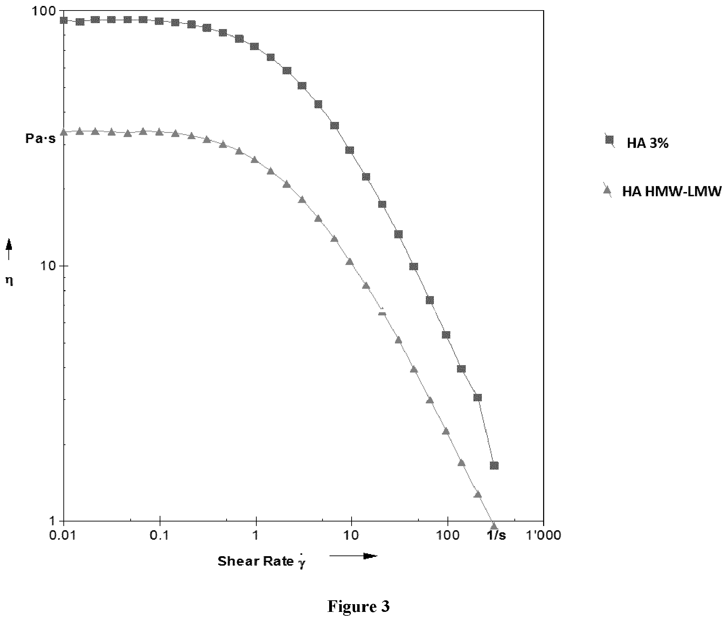

The solutions of Examples 2 and 3 are compared with a solution obtained by dissolving 30 g of sodium hyaluronate obtained by fermentation, having a molecular weight of 1,700 kDa, in 1 L of saline solution, and with a solution obtained by dissolving 21 g of sodium hyaluronate obtained by fermentation, having a molecular weight of 2,300 kDa, in 1 L of saline solution. Said two solutions, called HA 3% and HA 2.1% respectively, are analysed with a rheometer as described in Example 1. The graphs shown in Figures 2 (oscillatory analysis) and 3 (rotational analysis) compare HA HMW-LMW of Example 2 with HA 3% of Example 4.

The results are summarised in Table 4 below, which also shows the reference values for synovial fluid:

**Table 4**

| sample | crossover | G' and G" at crossover | dynamic viscosity (at 0.01 s⁻¹) |
|---|---|---|---|
| HA 3% | 9 rad/s | 115 Pa | 90 Pa*s |
| HA 2.1% | 6 rad/s | 95 Pa | 85 Pa*s |
| HA VHMW-LMW | 9 rad/s | 23 Pa | 12 Pa*s |
| HA HMW-LMW | 9 rad/s | 79 Pa | 20 Pa*s |
| HA VHMW-HMW-LMW | 9 rad/s | 40 Pa | 16 Pa*s |
| synovial fluid | 1-10 rad/s | 20-80 Pa | 1-40 Pa*s |

As will be seen, all five solutions have a crossover frequency comparable with that of synovial fluid. It is therefore possible to simulate the crossover frequency of the synovial fluid of a healthy individual using a single sodium hyaluronate at a more or less high concentration, depending on its molecular weight. However, said result is obtained at the expense of dynamic viscosity, which is more than twice that of the physiological value, and of the values of moduli G' and G" at the crossover point, which are significantly higher than those of synovial fluid.

Therefore, when a single hyaluronic acid is used, the rheological properties of the resulting solution are different from those of synovial fluid. Conversely, the same hyaluronate mixed with at least a second hyaluronate having a lower molecular weight gives rise to a marked improvement in viscoelastic behaviour, and the higher the molecular weight of the high-molecular-weight sodium hyaluronate obtained by fermentation, the more evident said improvement is.

### Example 5: influence of total concentration on the viscoelastic properties of a mixture of hyaluronates

A solution is prepared as described in Example 1 (16 g of sodium hyaluronate having a molecular weight of 1700 kDa mixed with 16 g of sodium hyaluronate having a molecular weight of 70 kDa dissolved in 1 L of saline solution). The solution has a total sodium hyaluronate concentration of 32 mg/ml, and exhibits behaviour comparable with that of healthy synovial fluid.

To simulate the preparations described in EP2026821, 4.4 g of saline solution is added to 2 g of the prepared solution, giving rise to a final concentration of 10 mg/ml. The values of G' and G" at the crossover frequency are determined as described above. The data obtained are summarised in Table 5 below.

**Table 5**

| sample | crossover | G' and G" at crossover | G' at 1 Hz | G" at 1 Hz |
|---|---|---|---|---|
| HA HMW-LMW 32 mg/ml | 9 rad/s | 79 Pa | 55 Pa | 66 Pa |
| HA HMW-LMW 10 mg/ml | > 100 rad/s | -- | 0.10 Pa | 0.95 Pa |
| synovial fluid | 1-10 rad/s | 20-80 Pa | n.d. | n.d. |

The preparation at 10 mg/ml no longer exhibits a crossover point, because the viscous component (G") always prevails over the elastic component (G') up to frequencies of 100 rad/s. It is therefore no longer comparable with healthy synovial fluid. Moreover, the values of G' and G", measured at 1 Hz, are much lower than those reported in EP2026821.

### Example 6: Evaluation of variation in rheological properties following addition of a phospholipid to a mixture of hyaluronates

1.6 g of phospholipids (phosphatidylcholine) is added to 1 L of the HA HMW-LMW solution of Example 2. The novel solution, called HA HMW-LMW + PC, undergoes rheological analysis as described in Example 2.

The results were compared with those of solution HA HMW-LMW of Example 2, and are summarised in Table 6 below, which also shows the reference values for synovial fluid:

**Table 6**

| sample | crossover | G' and G" at crossover | dynamic viscosity (at 0.01 s⁻¹) |
|---|---|---|---|
| HA HMW-LMW+ PC | 10 rad/s | 80 Pa | 23 Pa*s |
| HA HMW-LMW | 9 rad/s | 79 Pa | 20 Pa*s |
| synovial fluid | 1-10 rad/s | 20-80 Pa | 1-40 Pa*s |

The data demonstrate that the addition of phosphatidylcholine, at a concentration 20 times lower than that of sodium hyaluronate, does not give rise to any significant variations in the rheological properties of the preparation. As reported in the literature, the presence of phosphatidylcholine improves the lubrication capacity of the system.

### Example 7: comparative rheological evaluation of physical mixtures and complexes of hyaluronate

20 g of sodium hyaluronate obtained by fermentation with a molecular weight of 1,500 kDa, and 16 g of sodium hyaluronate obtained by fermentation with a molecular weight of 90 kDa, are dissolved in 1 L of saline solution. A 500 ml aliquot of the resulting solution "as is" undergoes rheological analysis. The remainder undergoes a cycle of heat treatment as described by WO 2012/032151 in Example 1, to form a hybrid cooperative complex. In particular, the solution is heated to reach a temperature of 118°C in 10 minutes, and said temperature is then maintained for 10 minutes. The solution is then cooled to 25°C in 10 minutes. The resulting solution is also examined with a rheometer.

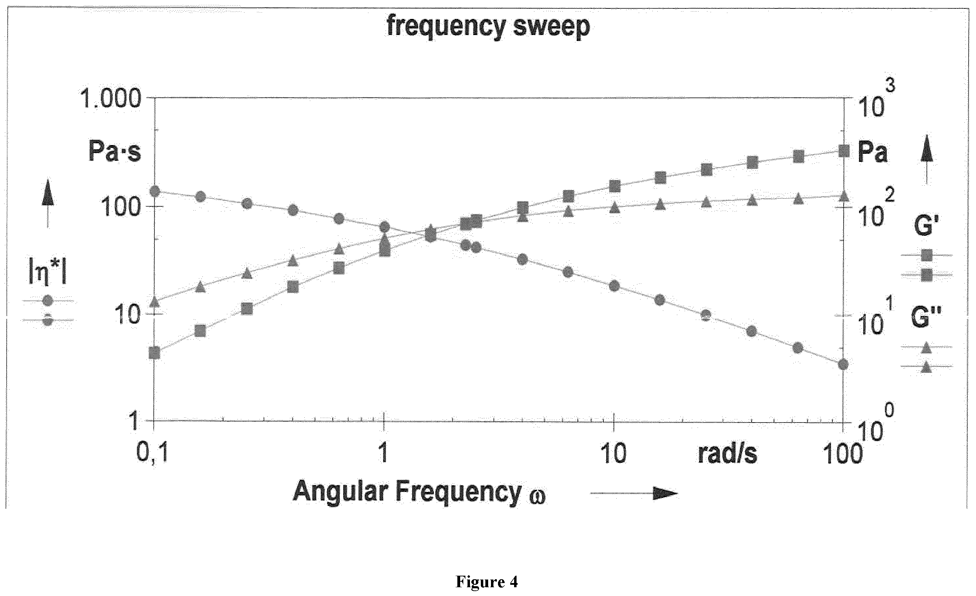

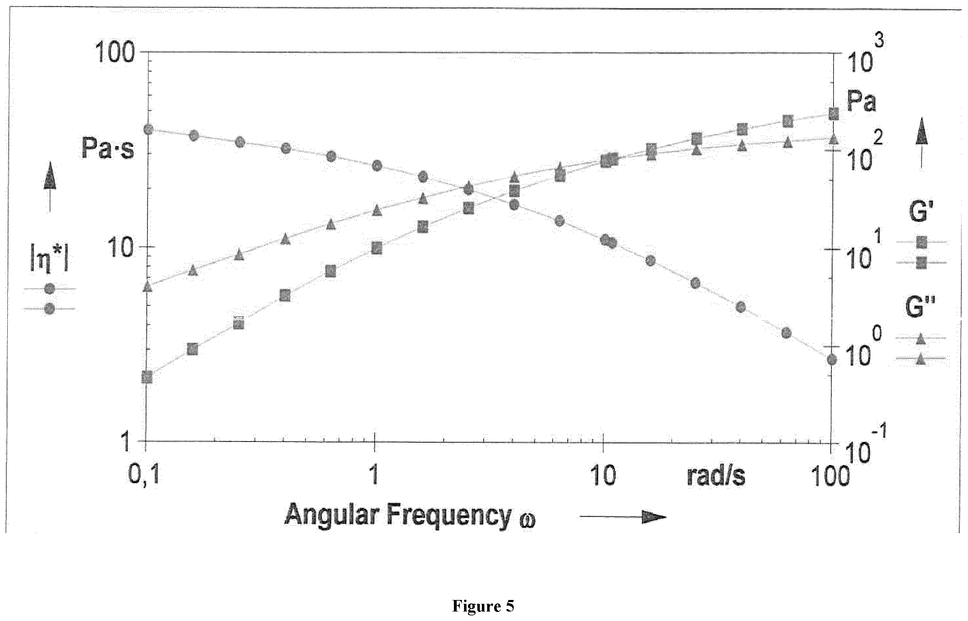

The graphs relating to the oscillatory analysis are shown in Figure 4 for the physical mixture of hyaluronates and Figure 5 for the corresponding complex. The main data are summarised in Table 7:

**Table 7**

| sample | crossover | G' and G" at crossover |
|---|---|---|
| HA physical mixture | 2.5 rad/s | 75 Pa |
| HA complex | 12 rad/s | 30 Pa |
| synovial fluid | 1-10 rad/s | 20-80 Pa |

Ingredients and concentration being equal, the hybrid cooperative complex of hyaluronic acid exhibits a significantly higher crossover frequency than the corresponding physical mixture. The formation of the complex therefore worsens the rheological properties of the mixture of hyaluronates, making the preparation less similar to synovial fluid and therefore less suitable for viscosupplementation.

## Claims

1. An intra-articular composition comprising at least one low molecular weight linear hyaluronic acid or hyaluronate having an average molecular weight Mw ranging from 70 to 90 kDa and at least one high molecular weight linear hyaluronic acid or hyaluronate having average molecular weight Mw ranging from 1000 to 3500 kDa, the weight ratio of low-molecular-weight hyaluronate to high-molecular-weight hyaluronate(s) ranging from 1:3 to 3:1, in concentrations of hyaluronic acids or hyaluronates ranging between 1 and 5% w/v, said composition having a dynamic viscosity at 25°C ranging from 10 to 60 Pa*s (at 0.01 s⁻¹). a crossover frequency ranging from 1 to 10 rad/s and a viscous and elastic modulus value at the crossover frequency ranging from 20 to 110 Pa.

2. A composition according to claim 1 comprising at least two different high molecular weight linear hyaluronic acids or hyaluronates.

3. A composition according to claims 1 or 2 further comprising a phospholipid.

4. A composition according to claim 3 wherein the phospholipid is phosphatidyl choline.

5. A composition according to one or more of claims 1 to 4 further comprising lubricin.

6. A composition according to one or more of claims 1 to 5 further comprising another glycosaminoglycan.

7. A composition according to claim 6 wherein the other glycosaminoglycan is non-sulphated chondroitin.

8. Compositions of claims 1-7 for use as a substitute for synovial fluid.

9. Compositions for use according to claim 8 for the treatment of joint disorders.

## Patentansprüche

1. Intraartikuläre Zusammensetzung, umfassend mindestens eine lineare Hyaluronsäure oder ein lineares Hyaluronat mit niedrigem Molekulargewicht und einem durchschnittlichen Molekulargewicht Mw im Bereich von 70 bis 90 kDa und mindestens eine lineare Hyaluronsäure oder ein lineares Hyaluronat mit hohem Molekulargewicht und einem durchschnittlichen Molekulargewicht Mw im Bereich von 1000 bis 3500 kDa,
wobei das Gewichtsverhältnis von Hyaluronat mit niedrigem Molekulargewicht zu Hyaluronat(en) mit hohem Molekulargewicht im Bereich von 1:3 bis 3:1, in Konzentrationen von Hyaluronsäuren oder Hyaluronaten im Bereich von 1 bis 5 Gew.-% liegt, wobei die Zusammensetzung eine dynamische Viskosität bei 25°C im Bereich von 10 bis 60 Pa*s (bei 0,01 s⁻¹), eine Übergangsfrequenz im Bereich von 1 bis 10 rad/s und einen Viskositäts- und Elastizitätsmodulwert bei der Übergangsfrequenz im Bereich von 20 bis 110 Pa aufweist.

2. Zusammensetzung nach Anspruch 1, umfassend mindestens zwei verschiedene lineare Hyaluronsäuren oder Hyaluronate mit hohem Molekulargewicht.

3. Zusammensetzung nach Anspruch 1 oder 2, ferner umfassend ein Phospholipid.

4. Zusammensetzung nach Anspruch 3, wobei das Phospholipid Phosphatidylcholin ist.

5. Zusammensetzung nach einem oder mehreren der Ansprüche 1 bis 4, ferner umfassend Lubricin.

6. Zusammensetzung nach einem oder mehreren der Ansprüche 1 bis 5, ferner umfassend ein weiteres Glykosaminoglykan.

7. Zusammensetzung nach Anspruch 6, wobei das andere Glykosaminoglykan nicht-sulfatiertes Chondroitin ist.

8. Zusammensetzungen nach den Ansprüchen 1 bis 7 zur Verwendung als Ersatz für Synovialflüssigkeit.

9. Zusammensetzungen zur Verwendung nach Anspruch 8 zur Behandlung von Gelenkerkrankungen.

## Revendications

1. Composition intra-articulaire comprenant au moins un acide hyaluronique ou hyaluronate linéaire de faible poids moléculaire présentant un poids moléculaire moyen Mw compris entre 70 et 90 kDa et au moins un acide hyaluronique ou hyaluronate linéaire de poids moléculaire élevé présentant un poids moléculaire moyen Mw compris entre 1 000 et 3 500 kDa, le rapport pondéral entre le hyaluronate de faible poids moléculaire et le(s) hyaluronate(s) de poids moléculaire élevé étant compris entre 1:3 et 3:1, en concentrations d'acides hyaluroniques ou hyaluronates comprises entre 1 et 5 % p/v, ladite composition présentant une viscosité dynamique à 25 °C comprise entre 10 et 60 Pa*s (à 0,01 s⁻¹), une fréquence de transition comprise entre 1 et 10 rad/s et une valeur de modules de viscosité et d'élasticité à la fréquence de transition comprise entre 20 et 110 Pa.

2. Composition selon la revendication 1 comprenant au moins deux acides hyaluroniques ou hyaluronates linéaires de poids moléculaire élevé différents.

3. Composition selon les revendications 1 ou 2, comprenant en outre un phospholipide.

4. Composition selon la revendication 3, dans laquelle le phospholipide est la phosphatidylcholine.

5. Composition selon une ou plusieurs des revendications 1 à 4, comprenant en outre de la lubricine.

6. Composition selon une ou plusieurs des revendications 1 à 5, comprenant en outre un autre glycosaminoglycane.

7. Composition selon la revendication 6, dans laquelle l'autre glycosaminoglycane est la chondroïtine non sulfatée.

8. Compositions selon les revendications 1 à 7, pour une utilisation comme substitut du liquide synovial.

9. Compositions pour une utilisation selon la revendication 8, pour le traitement de troubles articulaires.
